# EUROPEAN PATENT SPECIFICATION

(11) **EP 2 919 756 B1**
(45) Date of publication and mention of the grant of the patent: **28.11.2018**
(21) Application number: 13811630.6
(22) Date of filing: 13.11.2013
(51) Int. Cl.: A61K 9/00, A61K 9/10, A61K 31/00, A61K 8/00, A61K 47/14, A61P 17/14, A61P 17/10, A61K 9/51

(54) **SOLID LIPID NANOPARTICLES OF ROXITHROMYCIN FOR HAIR LOSS OR ACNE**
FESTE LIPIDNANOPARTIKEL AUS ROXITHROMYCIN FÜR HAARAUSFALL ODER AKNE
NANOPARTICULES LIPIDIQUES SOLIDES DE ROXITHROMYCINE POUR LA CHUTE DES CHEVEUX OU L'ACNÉ

(30) Priority: 14.11.2012 PL 40162512
(43) Date of publication of application: 23.09.2015
(73) Proprietor: Gdanski Uniwersytet Medyczny, 80-210 Gdansk (PL)
(72) Inventor: CAL, Krysztof, 80-289 Gdansk (PL); WOSICKA-FRACKOWIAK, Hanna, 61-493 Poznan (PL)
(74) Representative: Matyka, Malgorzata
(86) International application number: PCT/PL2013/000146
(87) International publication number: WO 2014/077712

(56) References cited:
- CA-A1- 2 483 786
- TAISUKE ITO ET AL: "Roxithromycin antagonizes catagen induction in murine and human hair follicles: implication of topical roxithromycin as hair restoration reagent", ARCHIVES OF DERMATOLOGICAL RESEARCH ; FOUNDED IN 1869 AS ARCHIV FÜR DERMATOLOGIE UND SYPHILIS, SPRINGER, BERLIN, DE, vol. 301, no. 5, 27 September 2008 (2008-09-27), pages 347-355, XP019713585, ISSN: 1432-069X cited in the application
- MÜNSTER U ET AL: "RU 58841-myristate--prodrug development for topical treatment of acne and androgenetic alopecia", JOURNAL OF ECONOMIC ENTOMOLOGY, ENTOMOLOGICAL SOCIETY OF AMERICA, LANDHAM, LANDHAM,MD,US, vol. 60, no. 1, 1 January 2005 (2005-01-01), pages 8-12, XP009098167, ISSN: 0022-0493 cited in the application
- ATRUX-TALLAU N ET AL: "Skin absorption modulation: Innovative non-hazardous technologies for topical formulations", OPEN DERMATOLOGY JOURNAL 2010 BENTHAM SCIENCE PUBLISHERS B.V. NLD, vol. 4, no. 1, 2010, pages 3-9, XP002719585, ISSN: 1874-3722 Retrieved from the Internet: URL:http://www.benthamscience.com/open/tod j/articles/V004/SI0001TODJ/3TODJ.pdf>
- WOSICKA H ET AL: "Targeting to the hair follicles: Current status and potential", JOURNAL OF DERMATOLOGICAL SCIENCE, ELSEVIER SCIENCE PUBLISHERS, SHANNON, IE, vol. 57, no. 2, 1 February 2010 (2010-02-01), pages 83-89, XP026874745, ISSN: 0923-1811 [retrieved on 2010-01-08]

## Description

### Field of invention

The invention concerns a pharmaceutical composition containing roxithromycin, the preparation method and medical application of the composition in the manufacturing of a medicinal product to treat androgenic hair loss and/or common acne, and the medicinal product containing the composition.

### Background art

Roxithromycinand its derivatives were first disclosed in patent EP 0 033 255 B applied for by ROUSSEL UCLAF.

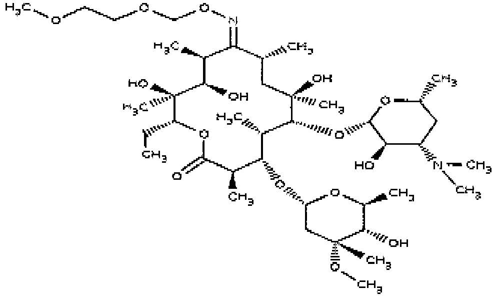

Known from the patent application CA 2 483 786 A preparation for treating androgenetic alopecia, hirsutism, i.e. the prevention of undesired hair growth, seborrhoea, and acne and can also be used in cosmetics but the active substance is not a roxithromycin.

Roxithromycin, an antibiotic of the semi-synthetic macrolide group, is a 10-oxime ether derivative of erythromycin of increased resistance to acids and better pharmacokinetic parameters. It is lipophilic in nature. Roxithromycin is administered orally in treating infections with Gram-positive bacteria, and, though less frequently, Gram-negative bacteria. The mechanism of roxithromycin's action involves blocking bacterial protein biosynthesis.

Roxithromycin, on top of its typical anti-bacterial action with respect to *Propionibacterium* acnes, inhibits the bacteria's production of pro-inflammatory compounds (lipase, NCF - neutrophil chemotactic factor) which also contribute to the development of acne. Thanks to the above, roxithromycin demonstrates very strong anti-inflammatory properties. There are reports on its inhibition of free radical formation in the neutrophils and on inhibition of keratinocyte apoptosis.

The basic causes of the development of the common acne are believed to include excessive sebum production, increased colonisation of the orifice area of the sebaceous gland duct by the Propionibacterium *acnes* bacteria, excessive keratinisation of the duct, and inflammation. Inflammation is primarily due to the said bacteria, and more specifically the free fatty acids formed in the process of decomposition of the sebum triglycerides by the bacterial lipases. Roxithromycin, due to its anti-bacterial action on *Propionibacterium* acnes and anti-inflammatory properties is the proper antibiotic in local treatment of acne.

The etiology of both the androgenic hair loss and common acne is connected with the pilosebaceous unit in the skin. The symptoms of the above diseases are difficult to conceal because of their location, and that is why they pose a grave problem to the patients, also in the psychological aspect, causing reduced self-esteem and deteriorated quality of life.

Both hair loss and acne are disorders which affect broad circles of the population. They weigh heavily on self-esteem and the related emotional state of the patients. Nowadays, when the looks play such a major role, new therapies treating the conditions are invaluable.

Minoxidil 2% and 5% is the only drug for local application accepted by the FDA as effectively counteracting androgenic hair lossin men and women. Finasteride is used in oral therapy of hair loss, however it carries grave side effects and can only be taken by men.

There are no alternative pharmaceuticals addressed specifically at hair loss. Noteworthy, there is no medicinal product for external administration containing roxithromycin that would be registered anywhere in the world.

Follicular penetration (into and through the hair follicles) of medicinal substances applied topically has for years been undervalued because of the minute area that hair follicles occupy compared to the area of the entire body. However, there are body areas where hair density is high enough for the follicles to offer a major drug penetration path, or for the targeted delivery to the specific sites in the follicle. The hair follicle density (500-1000/cm²) and the volume of the follicular opening on the forehead and head are the highest on the human body. The combined area of the follicular openings in these locations can be as much as 10% of the total skin area in these body regions. The same body sites frequently suffer from dermatological disorders such as androgenic hair loss and common acne, the etiology of which is closely related to the pilosebaceous apparatus. In such cases targeted drug delivery to the hair follicles becomes the key element of the therapy.

Apart from the hair follicle with the hair shaft, the structure of the pilosebaceous unit includes the sebaceous gland that opens into the canal, and the arrector pili muscle. The hair shaft can be divided into three zones called (top-down): *infundibulum, isthmus,* and the suprabulbar region ending with a hair bulb. The first two parts extend at the epidermal and dermal levels, whereas the third section of the hair shaft together with the bulb can be located as far as the subcutaneous tissue. The opening of the sebaceous gland duct to the hair canal is located at the border of the *infundibulum* and *isthmus* zones. On the other hand, at the border of the second and third zones of the hair shaftis the so-called bulge region containing stem cells which participate in hair reconstruction over the hair cycle. This is also the region where the arrector pili muscle is attached.

The hair shaft is enclosed in two sheaths: outer and inner. The outer hair root sheath is continuous with the epidermis of the skin invaginating into the dermis. The sebaceous gland is an element attached to the outer root sheath. The gland is formed from sebocytes -large cells producing and storing the sebum, and keratinocytes - forming the gland opening into the hair canal. There are several regions in the pilosebaceous apparatus where targeted administration of substances for therapeutic purposes is both possible and beneficial, for instance the so-called bulge region with stem cells - the object of interest to gene therapy. The sebaceous glands are also a promising target area, as they open into the hair follicles and at the same time are the location where the *Propionibacterium* acnes develop being one of the contributors to the common acne. The largest and most densely spread sebaceous glands are observed on the face and head. Mature sebocytes secrete sebum in the holocrine process (cell disintegration). The sebum is a mixture of triglycerides, waxes, squalene, fatty acids, and cholesterol. The sebum forms the first barrier to be overcome by any substances applied topically to the mentioned body areas. On the other hand, thanks to its lipophilic nature it does not hinder penetration of substances of the same properties.

The sebaceous glands are related to the pathogenesis of not only the common acne, but also androgenic hair loss. It is in them, where the expression of 5a-reductase takes place, the 5a-reductase being the enzyme responsible for testosterone transformation to 5a-dihydrotestosterone, which in turn causes miniaturisation of the sensitive hair follicles and accelerates hair loss.

### Summary of the invention

In androgenic hair loss, dihydrotestosterone causes apoptosis of the sensitive cells of the hair papilla accelerating the passage from the anagen to the catagen phase. Roxithromycin, considering e.g. its anti-apoptotic action seems to be the proper candidate to inhibit the process. In the in *vitro*tests with human hair follicles roxithromycin was observed to inhibit the follicle's passage to the catagen phase through inhibition of keratinocyte apoptosis and as a result it caused hair elongation. In the *in* vivo study, on the other hand, restored hair growth was observed in more than half of the patient population suffering from androgenic hair loss after external application of 0.5% solution of roxithromycin for 24 weeks. Substantial thickening of the hair was also noted. Moreover, no local or systemic side effects or undesirable action was recorded.

The lipid microparticles and nanoparticles are the proper drug carrier for roxithromycin in corporation due to its lipophilic nature. Their characteristic features are: low toxicity and biodegradation. Another of their important advantages is the fact that they protect the incorporated drug from chemical degradation. Thanks to their lipophilic nature, they are suitable carriers for targeted drug delivery to the pilosebaceous unit. They have been proved to reach human hair follicles effectively. Accumulating there, they form a reservoir of the incorporated drug and ensure that its release and action extends to 10 days. The microparticles and nanoparticles' ability to penetrate the follicles is related to their size. The diameter of the follicular openings in the forehead region is ca. 60 µm, and the diameter of the hair shaftitself ranges about 15 µm. Until recently it was believed that the sebum flowing out of the hair canal prevents or seriously hinders penetration of active substances into the follicles. However, considering the very slow rate at which the sebum seeps out of the sebaceous gland and onto the skin surface, one can conclude that it does not inhibit formulation penetration into the hair follicles. The latter has been confirmed in the tests where the particulate carriers were found present in the hair canal as soon as 15 min after the application.

It has been proved that a drug incorporated in a particulate carrier reaches hair follicles in larger quantities than a drug applied e.g. in the form of a solution. The application method plays an important role in follicular penetration by drug carriers - skin massage substantially increases the penetration depth. Formulation application preceded with superficial exfoliation (removal of the external layers of *stratum* corneum) also improves penetration of the hair follicles by drug carriers. Effective penetration of particulate carriers into the follicles is closely related to the particle size. Particles > 10 µm remain on the skin surface, whereas those of a lesser diameter penetrate the hair canal, and the smaller the diameter, the deeper they penetrate it.

Hence, targeted drug delivery to the pilosebaceous unit using particulate carriers of a specific size is possible. For instance, solid lipid nanoparticles about 500 nm in diameter whose penetration into the skin has been studied on the human scalp skin, were observed in hair follicles as deep as 900 µm. Their presence was also reported in the sebaceous glands.

The available literature data indicate that roxithromycin has not been incorporated into the lipid microparticles and nanoparticles. No studies either have been conducted to test roxithromycin penetration into and through the skin, including hair follicles, upon topical administration. Nowhere in the world is there any registered preparation containing the antibiotic that would be designated for topical application.

The composition according to the invention takes the form of an aqueous suspension of lipid microspheres.

Studies have evidenced that the products containing the composition according to the invention demonstrate very good follicular penetration properties.

The unexpected effects of applying the composition according to the invention are the outcome of a specific selection of the components and the production method:
- Glyceryl behenate (commercially available under the name Compritol 888 ATO) is the glyceride being the material which forms the microspheres (matrix).
- Poloxamer (commercially available under the name Poloxamer 188) plays the role of the emulsifier, which enables the formation of the microspheres.

The composition according to the invention may be used as a component of medicinal products.Typically, the composition according to the invention accounts for 1.0-100% w/w of the total weight of the medicinal product.

The particularly preferable in form are medicinal products designated for the face, head, and hair, and the composition finds its most preferable application in anti-acne and anti-alopecia preparations, e.g. creams, shampoos, lotions, fluids, and suspensions.

The composition according to the invention can be produced in a process comprising the following stages:
- heating the lipid and aqueous phases in separate vessels,
- combining the heated components,
- homogenising the mixture,
- cooling the mixture, and
- optionally - adding preservatives

The heating is conducted using standard techniques within the range of 75-85°C. Homogenisation of the mixture can be carried out under any technique used in the industry. For instance, without limiting the scope of the invention, it is possible to apply high-speed/high shear homogenisation, ultrasound homogenisation (sonication), or high-pressure homogenisation. The homogenisation stage, ending with thorough breaking up and perfect blending of the mixture components, assures e.g. stability and proper consistency of the ultimate suspension.

High-speed/high shear homogenisation involves subjecting the mixture to mechanical forces in effect of intense and high speed mixing. According to the invention, homogenisation employing the technique can preferably be carried out at the speed of 20 TH rpm for the time of 15 min.

Ultrasound homogenisation (sonication) takes advantage of the cavitation phenomenon. With the mixture exposed to intense ultra sounds, the sound waves spreading in it generate currents and whirls which in turn break the agglomerates to fine particles.

High-pressure homogenisation comes down to pumping the mixture through a specially constructed valve of a tiny but regulated lumen. The pressures needed in the process may reach 20 - 150 MPa.

Mixture cooling brings it down to the room temperature, preferably in the range of 15-25°C. By the reduction of the mixture's temperature the process of lipid solidification and formation of solid microspheres is accomplished.

After cooling, and if necessary, it is possible to add preservatives to the mixture.
The gist of the invention is the pharmaceutical composition containing roxithromycin, composed as follows:
a. 1.0-40.0% solid lipids: glyceryl mono-,di- and tri- behenate;
b. 0.1-10.0% roxithromycin
c. 1.0-10.0% surfactants: poloxamer 188, and
d. optional 0-20.0% of preservatives, and
e. complementing water to make 100%.
The pharmaceutical composition according to another embodiment of the invention contains:
a. 5.0% glyceryl behenate
b. 1.0% roxithromycin
c. 2.5% poloxamer 188
d. 91.5% water
The pharmaceutical composition according to another embodiment of the invention contains:
a. 5.0% glyceryl behenate
b. 0.5% roxithromycin
c. 2.5% poloxamer 188
d. 92.0% water

The pharmaceutical composition where the solid lipids are glyceryl mono-, di- and tri- behenate. Other solid lipids may be glyceryl mono- di- or tri- myristate. glyceryl palmitate or stearate; cetyl palmitate; stearic acid; palmitic acid, or a mixture of the listed substances.

The pharmaceutical composition where the surfactants are poloxamer 188. Other surfactants may be poloxamers or other non-ionic surfactants.

The pharmaceutical composition where poloxamer 188 is selected out of the poloxamer group.

The pharmaceutical composition where the preservatives are selected from the group including phenoxyethanol, glycols, or glycerine derivatives.

The pharmaceutical composition where preferably the content of preservatives is at the weight proportion of: 0-2.0% for phenoxyethanol and/or 0-20.0% for pentylene or butylene glycol, or glycerine derivatives.

The pharmaceutical composition which contains a lipid phase in the form of lipid microspheres at the weight proportion of 1.0 - 40.0%, preferably at the weight proportion of 5.0 - 20.0%.

The pharmaceutical composition which contains a lipid phase in the form of lipid microspheres sized 0.1 to 1.0 µm in diameter, preferably 0.1 to 0.5 µm.

The application of the pharmaceutical composition, where the composition is used to produce medicinal products targeted at treating androgenic hair loss and/orcommon acne, in any pharmaceutical form including lotions, fluids, oil-in-water emulsions, creams, gels, shampoos, balms, conditioners, soaps, ointments, pastes, suspensions, and solutions.

The medicinal product which contains the composition defined in any of the claims 1-3 in the weight proportion of 1.0-100%.

The medicinal product is selected from the group including lotions, fluids, oil-in-water emulsions, creams, gels, shampoos, balms, conditioners, soaps, ointments, pastes, suspensions, and solutions.

The medicinal product is selected from the group including cream, shampoo, suspension, and lotion.

The preparation method of the pharmaceutical composition comprising the following stages:
a) the aqueous phase and components of the lipid phase are heated separate to the temperature of 75-85°C,
b) both phases are combined and homogenised, and
c) the obtained microemulsion is cooled down to the room temperature,
d) after the cooling, preservatives are added.

The method where homogenisation at stage b) is conducted applying any technique selected from the group including high-speed/high shear homogenisation, ultrasound homogenisation and/or high-pressure homogenisation.

### Figure legend:

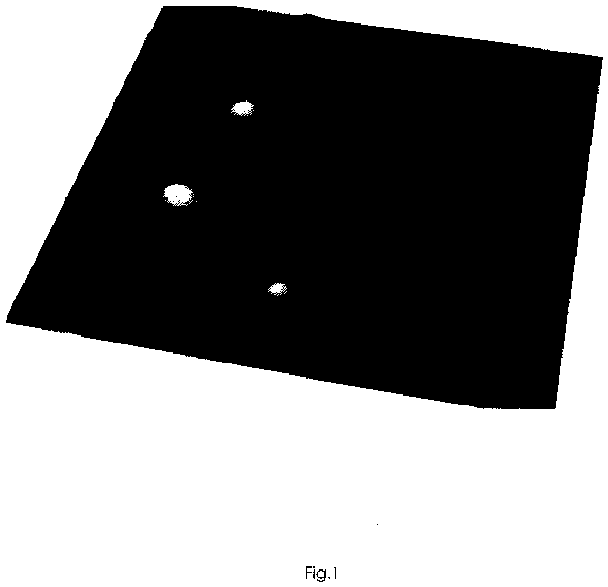

Fig. **1****.** an AFM view of the microspheres produced according to example 1.

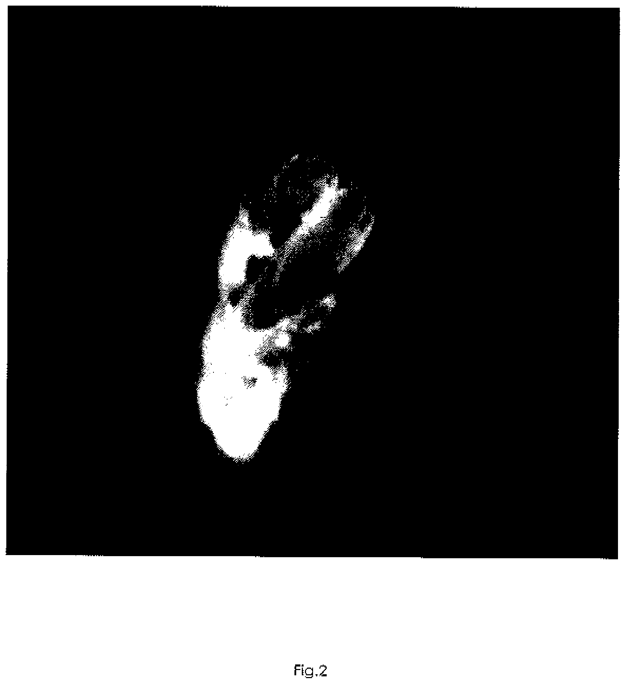

Fig.2 microsphere penetration (according to example 1) into the hair follicles to the depth of 1.5 mm (test according to example 7).

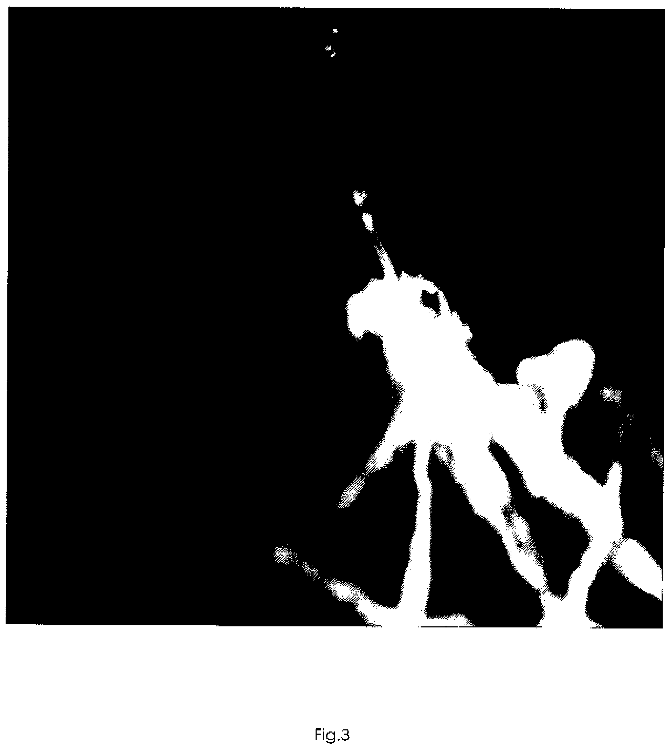

Fig.3 biopsy of the hair follicle content (according to example7).

The invention is illustrated with the following examples, none of which imposes any restriction thereon

### Example 1

The aqueous phase (91.5% water, and 2.5% poloxamer 188) and the lipid phase (5.0% glyceryl behenate, and 1.0% roxithromycin), where all per cent values express the ratio to the total weight of the composition, were heated in separate vessels to the temperature of about 80°C. Then, the aqueous phase and the lipid phase were combined and instantly subjected to high-speed homogenisation (20 TH rpm, 15 min). The obtained microemulsion was cooled down to the room temperature (15-25°C), which caused lipid solidification and formation of solid microspheres.

The composition obtained contained the following components (% w/w figures):

| | |
|---|---|
| 5.0% | glyceryl behenate |
| 2.5% | poloxamer 188 |
| 1.0% | roxithromycin |
| 91.5% | water |

### Example 2

The aqueous phase (92.0% water, and 2.5% poloxamer 188) and the lipid phase (5.0% glyceryl behenate, and 0.5% roxithromycin), where all per cent values express the ratio to the total weight of the composition, were heated in separate vessels to the temperature of about 80°C. Then, the aqueous phase and the lipid phase were combined and instantly subjected to high-speed homogenisation (20 TH rpm, 15 min). The obtained microemulsion was cooled down to the room temperature (15-25°C), which caused lipid solidification and formation of solid microspheres.

The composition obtained contained the following components (%w/w figures):

| | |
|---|---|
| 5.0% | glyceryl behenate |
| 2.5% | poloxamer 188 |
| 0.5% | roxithromycin |
| 92.0% | water |

### Example 3

The physical and chemical features of the composition in example 1 were tested.
- Shape of the lipid particles in the composition: spherical.
   (measuring techniques - SEM imaging (scanning electron microscopy), TEM (transmission electron microscopy), AFM (atomic force microscopy).
- Size and diameter of the particles: ca. 200 nm.
   (measuring technique - DLS (dynamic light scattering)
- Zeta potential: -20 - -50 mV.
   (measuring technique -laser doppler electrophoresis). Polydispersity index (PDI): less than 0.30.
- pH of the composition: 7.0-9.0.
- Roxithromycin incorporation rate in the particles: ca. 80% (HPLC method).

### Example 4

The composition obtained in example 1 was subjected to thermal analysis (DSC). The test revealed only one endothermic peak at the temperature of ca. 74°C, which indicates that it corresponds to the lipid matrix melting. On the other hand, there is no peak at the temperature of ca. 125°C, which would indicate roxithromycin melting. This means that roxithromycinis dissolved in the lipid matrix.

### Example 5

The composition obtained in example 1 was subjected to a test aimed at verifying its antibacterial properties with respect to *Propionibacterium* acnes. The test was conducted in compliance with the Ph. Eur. 7.0 guidelines for 3 formulations: placebo, and formulations containing 0.5% and 1% of roxithromycin. In the case of the placebo formulation no inhibition of the microbe growth was observed. On the other hand, in the case of the other two formulations the *Propionibacterium* acnes growth rate was observed to be reduced by 99.9%.

### Example 6

The composition obtained in example 1 was subjected to a test aimed at establishing its cytotoxicity. The test was conducted on the human epidermis model, *Epiderm*, and comprised 3 formulations: placebo, and formulations containing 0.5% and 1% of roxithromycin. Cell survival on application of the placebo preparation was ca. 82%, whereas upon application of the roxithromycin-containing formulations - ca. 93%.

### Example 7

The composition obtained in example 1 was subjected to a test aimed at visualizing the penetration of the lipid particles with roxithromycin into the hair follicles in ex *vivo*conditions. The formulation was produced with an admixture of a fluorescent dye, namely rhodamine B hexyl ester, and oil solution of the same dye was used as the control. The test was conducted on ex vivo samples of human scalp and chest skin. Application of the preparation to the skin was massager-assisted. Upon incubation, vertical and horizontal sections of the frozen skin samples were taken and examined on the spot under a fluorescent microscope.

The images obtained evidenced that the applied particle formulation accumulates around the hair follicles (fluorescence visible deep into the follicle and down to the hair bulb). The photographs of the skin cross-sections reveal fluorescence at the depth of more than 1 mm. Upon application of the dye solution very strong fluorescence is visible on the skin surface, whereas there is no trace of fluorescence in the hair canal. This proves that massage-assisted application of the particulate carrier is capable to deliver the drug deep into the hair follicle, and most of all can do so selectively.

The chest skin fragment was also subjected to the *differential stripping procedure.* First, the sample was tape *stripped* to remove 10 fractions of *stratum* corneum. Then a drop of cyano acrylate glue was applied and the microscope slide on top. Once the glue has polymerised, the slide was detached together with the thus removed follicular content then examined under the fluorescent microscope. The biopsies showing the hair shaft removed together with the follicular content proved fluorescent, also on the hair surface. This evidenced the particulate carrier penetration path in the follicles and proved that the formulation penetrated deep into the hair canal.

### Example 8

The composition obtained in example 1 was subjected to a test aimed at verifying follicular penetration of the lipid particles with roxithromycin in the in *vivo* conditions and assessing the scale of the process.

The test was conducted *in vivo* on skin fragments in the fore arm and shin areas. The skin of the sites was washed, dried, and the hair clipped with scissors. 6 fragments, 5 cm² each, were isolated in each of the above indicated areas. Their edges were contoured and safe guarded with tape. 100 µl of the composition defined in example 1 was applied to each of the 5 cm² areas, then massaged for 5 minutes using the Atom Massager (HoMedicsGroup, Tonbridge. United Kingdom). Upon the lapse of 30 min or 12 h the *stratum corneum* was *tape-*stripped (to remove the composition present on the skin surface) and then the skin was subjected to *differential stripping.*

Cyanoacrylic glue was applied in 4 drops per each of the 5 cm² areas. The obtained cyanoacrylic biopsies containing follicular content were examined further to identify the amount of roxithromycin. Each biopsy (5 cm²) was extracted with 5 ml of methanol which was then subjected to assay under the high performance liquid chromatography technique combined with mass spectrometry (HPLC - MS).

The obtained results are presented in the table below:

| **Biopsy site** | **Formulation penetration time (time between the massaging and the differential stripping)** | **Average roxithromycincontent in the biopsy (n=3) [µg]** | **Average roxithrormycicontent per 1cm²[µg/cm²]** |
|---|---|---|---|
| forearm | 30 min | 15.2 | 3.0 |
| forearm | 12 h | 23.7 | 4.7 |
| shin | 30 min | 19.3 | 3.9 |
| shin | 12 h | 2.4 | 0.5 |

### Example 9 (not the invention)

Alternative compositions made up of the following components (%w/w proportions):
a)

| | |
|---|---|
| Hydrogenated palm oil (Softisan 154) | 5.0% |
| Poloxamer 188 | 2.5% |
| Roxithromycin | 1.0% |
| Phenoxyethanol | 1.0% |
| Water | 90.5% |

b)

| | |
|---|---|
| Glyceryl behenate | 5-0% |
| Polyglyceryl-3 methylglucose distearate(Tego Care 450) | 2.5% |
| Roxithromycin | 1.0% |
| Water | 91.5% |

c)

| | |
|---|---|
| Hydrogenated palm oil (Softisan 154) | 5.0% |
| Poloxamer 188 | 2.5% |
| Roxithromycin | 1.0% |
| Water | 91.5% |

### References:

1. Akamatsu H, Tomita T, Horio T. Effects of roxithromycin on the production of lipase and neutrophil chemotactic factor by Propionibacterium acnes. Dermatology 2002; 204: 277-280
2. Downie MMT, Guy R, Kealey T. Advances in sebaceous gland research potential: New approaches to acne management. Int J Cosmetic Sci 2004; 26: 291-311
3. Hordinsky M. Advances in hair diseases. Adv Dermatol 2008; 24: 245-259
4. Ito T, Fukamizu H, Ito N, Seo N, Yagi H, Takigawa M, Hashizume H. Roxithromycin antagonizes catagen induction in murine and human hair follicles: implication of topical roxithromycin as hair restoration reagent. Arch Dermatol Res 2009; 301: 347-355
5. Knorr F, Lademann J, Patzelt A, Sterry W, Blume-Peytavi U, Vogt A. Follicular transport route - research progress and future perspectives. Eur J Pharm Biopharm 2009; 71: 173-180
6. Kobayashi M, Kabashima K, Nakamura M, Tokura Y. Effects of oral antibiotic roxithromycin on quality of life in acne patients. J Dermatol Sci 2009; 36: 383-391
7. Krause K, Foitzik K. Biology of the hair follicle: The basics. Semin Cutan Med Surg 2006; 25: 2-10
8. Lademann J, Richter H, Schaefer UF, Blume-Peytavi U, Teichmann A, Otberg N. Hair follicles - a long-term reservoir for drug delivery. Skin Pharmacol Physiol 2006; 19: 232-236
9. Munster U, Nakamura C, Haberland A, Jores K, Mehnert W, Rummel S, Schaller M, Korting HC, Zouboulis CC, Blume-Peytavi U, Schafer-Korting M. RU 58841-myristate - prodrug development for topical treatment of acne and androgenetic alopecia. Pharmazie 2005; 60: 8-12
10. Otberg N, Richter H, Schaefer H, Blume-Peytavi U, Sterry W, Lademann J. Variations of hair follicle size and distribution in different body sites. J Invest Dermatol 2004; 122: 14-19
11. Takahashi H, Suzuki Y, Miyauchi Y, Hashimoto Y, Ishida-Yamamoto A, lizuka H. Roxithromycin decreases ultraviolet B irradiation-induced reactive oxygen intermediates production and apoptosis of keratinocytes. J Dermatol Sci 2004; 34: 25-33
12. Teichmann A, Jacobi U, Ossadnik M, Richter H, Koch S, Sterry W, Lademann J. Differential stripping: determination of the amount of topically applied substances penetrated into the hair follicles, J Invest Dermatol 2005; 125: 264-269
13. Thiboutot D. Regulation of human sebaceous glands. J Invest Dermatol 2004; 123: 1-12
14. Trauer S, Lademann J, Knorr F, Richter H, Liebsch M, Rozycki C, Balizs G, Buttermeyer R, Linscheid M, Patzelt. Development of an in vitro modified skin absorption test for the investigation of the follicular penetration pathway of caffeine. Skin Pharmacol Physiol 2010; 23: 320-327
15. Wang KC, Zane LT. Recent advances in acne vulgaris research: Insights and clinical implications. Adv Dermatol 2008; 24: 197-209
16. Wei Lu G, Valiveti S, Spence J, Zhuang C, Robosky L, Wade K, Love A, Hu L, Pole D, Mollan M. Comparison of artificial sebum with human and hamster sebum samples. Int J Pharm 2009; 367: 37-43
17. Wosicka H, Cal K. Targeting to the hair follicles: Current status and potential. J Dermatol Sci 2010; 57: 83-89

## Claims

1. Pharmaceutical composition containing roxithromycin, **characterised in that** it is composed as follows:
a. 1.0-40.0% solid lipids: glyceryl mono-, di- and tri- behenate;
b. 0.1-10.0% roxithromycin
c. 1.0-10.0% surfactants: poloxamer 188
and
d. optional 0-20.0% of preservatives, and
e. complementing water to make 100%.

2. The pharmaceutical composition according to Claim 1, **characterised in that** it contains a lipid phase and is composed as follows:
a. 5.0% glyceryl behenate
b. 1.0% roxithromycin
c. 2.5% poloxamer 188
and
d. 91.5% water

3. The pharmaceutical composition according to Claim 1, **characterised in that** it contains a lipid phase and is composed as follows:
a. 5.0% glyceryl behenate
b. 0.5% roxithromycin
c. 2.5% poloxamer 188
and
d. 92.0% water

4. The pharmaceutical composition according to Claim 1, **characterised in that** the preservatives are selected from the group including phenoxyethanol, or glycerine derivatives.

5. The pharmaceutical composition according to Claim 4, **characterised in that** preferably the content of preservatives is at the weight proportion of: 0-2.0 % for phenoxyethanol and/or 0-20.0% for pentylene or butylene glycol, or glycerine derivatives.

6. The pharmaceutical composition according to Claims 1-5, **characterised in that** it contains a lipid phase in the form of lipid microspheres at the weight proportion of 1.0 - 40.0%, preferably at the weight proportion of 5.0 - 20.0%.

7. The pharmaceutical composition according to Claims 1-6, **characterised in that** it contains a lipid phase in the form of lipid microspheres sized 0.1 to 1.0 µm in diameter, preferably 0.1 to 0.5 µm.

8. A pharmaceutical composition according to claims 1- 7 for use in the treatment of androgenic hair loss and/or common acne, wherin the pharmaceutical composition is in any form including lotions, fluid, oil-in-water emulsions, creams, gels, shampoos, balms, conditioners, soaps, ointments, pastes, suspensions, and solutions.

9. The medicinal product **characterised in that** it contains the composition defined in any of the Claims 1-3 in the weight proportion of 1.0-100%.

10. The medicinal product according to Claim 9, **characterised in that** it is selected from the group including lotions, fluids, oil-in-water emulsions, creams, gels, shampoos, balms, conditioners, soaps, ointments, pastes, suspensions, and solutions.

11. The medicinal product according to Claim 10, **characterised in that** it is selected from the group including cream, shampoo, suspension, and lotion.

12. The preparation method of the pharmaceutical composition according to Claims 1-3, **characterised in that** it comprises the following stages:
a) the aqueous phase and components of the lipid phase are heated separate to the temperature of 75-85°C.
b) both phases are combined and homogenised, and
c) the obtained microemulsion is cooled down to the room temperature,
d) after the cooling, preservatives are added.

13. The method according to Claim 12, **characterised in that** homogenisation at stage b) is conducted applying any technique selected from the group including high-speed/high shear homogenisation, ultrasound homogenisation and/or high-pressure homogenisation.

## Patentansprüche

1. Pharmazeutische Zusammensetzung enthaltend Roxithromycin, **dadurch gekennzeichnet, dass** sie wie folgt zusammengesetzt ist:
a. 1,0 - 40,0 % feste Lipide: Glycerylmono-, -di- und -tribehenat;
b. 0,1 - 10,0 % Roxithromycin
c. 1,0 - 10,0 % Tenside: Poloxamer 188
und
d. optional 0 - 20,0 % Konservierungsstoffe und
e. ergänzend Wasser, um 100 % zu erhalten.

2. Pharmazeutische Zusammensetzung nach Anspruch 1, **dadurch gekennzeichnet, dass** sie eine Lipidphase enthält und wie folgt zusammengesetzt ist:
a. 5,0 % Glycerinbehenat
b. 1,0 % Roxithromycin
c. 2,5 % Poloxamer 188
und
d. 91,5 % Wasser

3. Pharmazeutische Zusammensetzung nach Anspruch 1, **dadurch gekennzeichnet, dass** sie eine Lipidphase enthält und wie folgt zusammengesetzt ist:
a. 5,0 % Glycerinbehenat
b. 0,5% Roxithromycin
c. 2,5 % Poloxamer 188
und
d. 92,0 % Wasser

4. Pharmazeutische Zusammensetzung nach Anspruch 1, **dadurch gekennzeichnet, dass** die Konservierungsstoffe aus der Gruppe umfassend Phenoxyethanol oder Glycerinderivate ausgewählt werden.

5. Pharmazeutische Zusammensetzung nach Anspruch 4, **dadurch gekennzeichnet, dass** der Gehalt an Konservierungsmitteln vorzugsweise bei einem Gewichtsanteil von 0 - 2,0 % für Phenoxyethanol und/oder 0 - 20,0 % für Pentylen- oder Butylenglykol oder Glycerin derivate liegt.

6. Pharmazeutische Zusammensetzung nach Anspruch 1 bis 5, **dadurch gekennzeichnet, dass** sie eine Lipidphase in Form von Lipid-Mikrosphären im Gewichtsanteil von 1,0 - 40,0 %, vorzugsweise im Gewichtsanteil von 5,0 - 20,0 %, enthält.

7. Pharmazeutische Zusammensetzung nach Anspruch 1 bis 6, **dadurch gekennzeichnet, dass** sie eine Lipidphase in Form von Lipid-Mikrosphären mit einem Durchmesser von 0,1 bis 1,0 µm, vorzugsweise 0,1 bis 0,5 µm, enthält.

8. Eine pharmazeutische Zusammensetzung nach Anspruch 1 bis 7 zur Verwendung bei der Behandlung von androgenem Haarausfall und/oder gewöhnlicher Akne, wobei die pharmazeutische Zusammensetzung in beliebiger Form vorliegt, darunter Lotionen, Flüssigkeit, Öl-in-Wasser-Emulsionen, Cremes, Gele, Shampoos, Balsame, Conditioner, Seifen, Salben, Pasten, Suspensionen und Lösungen.

9. Arzneimittel, **dadurch gekennzeichnet, dass** es die in einem der Ansprüche 1 - 3 definierte Zusammensetzung im Gewichtsanteil von 1,0 - 100 % enthält.

10. Arzneimittel nach Anspruch 9, **dadurch gekennzeichnet, dass** es aus der Gruppe umfassend Lotionen, Flüssigkeiten, Öl-in-Wasser-Emulsionen, Cremes, Gele, Shampoos, Balsame, Conditioner, Seifen, Salben, Pasten, Suspensionen und Lösungen ausgewählt ist

11. Arzneimittel nach Anspruch 10, **dadurch gekennzeichnet, dass** es aus der Gruppe umfassend Creme, Shampoo, Suspension und Lotion ausgewählt ist.

12. Verfahren zur Herstellung der pharmazeutischen Zusammensetzung nach Anspruch 1 - 3, **dadurch gekennzeichnet, dass** es die folgenden Stufen umfasst:
a) die wässrige Phase und die Bestandteile der Lipidphase werden getrennt auf die Temperatur von 75 - 85 °C erwärmt,
b) beide Phasen werden kombiniert und homogenisiert und
c) die erhaltene Mikroemulsion wird auf Raumtemperatur abgekühlt,
d) nach dem Abkühlen werden Konservierungsstoffe zugesetzt.

13. Verfahren nach Anspruch 12, **dadurch gekennzeichnet, dass** die Homogenisierung in Stufe b) unter Anwendung einer beliebigen Technik aus der Gruppe umfassend Hochgeschwindigkeits-/Hochscher-Homogenisierung, Ultraschall- und/oder Hochdruckhomogenisierung durchgeführt wird.

## Revendications

1. Composition pharmaceutique contenant de la roxithromycine, **caractérisée en ce qu'elle** se compose comme suit :
a. 1,0-40,0% de lipides solides : mono-, di- et tri-béhénate de glycéryle ;
b. 0,1-10,0% de roxithromycine
c. 1,0-10,0% d'agents tensioactifs : poloxamère 188
et
d. optionnellement 0-20,0% de conservateurs, et
e. un complément d'eau pour atteindre les 100%.

2. Composition pharmaceutique suivant la revendication 1, **caractérisée en ce qu'elle** contient une phase lipidique et se compose comme suit :
a. 5,0% de béhénate de glycéryle
b. 1.0% de roxithromycine
c. 2,5% de poloxamère 188
et
d. 91,5% d'eau

3. Composition pharmaceutique suivant la revendication 1, **caractérisée en ce qu'elle** contient une phase lipidique et se compose comme suit :
a. 5,0% de béhénate de glycéryle
b. 0,5% de roxithromycine
c. 2,5% de poloxamère 188
et
d. 92,0% d'eau

4. Composition pharmaceutique suivant la revendication 1, **caractérisée en ce que** les conservateurs sont choisis dans le groupe comprenant le phénoxyéthanol ou les dérivés de la glycérine.

5. Composition pharmaceutique suivant la revendication 4, **caractérisée en ce que** la teneur en conservateurs est de préférence en proportion pondérale de : 0-2,0% pour le phénoxyéthanol et/ou de 0-20,0% pour le pentylène ou le butylène glycol ou les dérivés de la glycérine.

6. Composition pharmaceutique suivant les revendications 1-5, **caractérisée en ce qu'elle** contient une phase lipidique sous forme de microsphères lipidiques en proportion pondérale de 1,0-40,0%, de préférence en proportion pondérale de 5,0- 20,0%.

7. Composition pharmaceutique suivant les revendications 1-6, **caractérisée en ce qu'elle** contient une phase lipidique sous la forme de microsphères lipidiques ayant un diamètre de 0,1 à 1,0 µm, de préférence de 0,1 à 0,5 µm.

8. Composition pharmaceutique suivant les revendications 1-7 pour l'utilisation dans le traitement d'alopécie androgénique et/ou de l'acné commune, dans laquelle la composition pharmaceutique prend l'une quelconque des formes suivantes : lotions, fluide, émulsions huile-dans-eau, crèmes, gels, shampooings, baumes, conditionneurs, savons, pommades, pâtes, suspensions et des solutions.

9. Médicament **caractérisé en ce qu'il** contient la composition définie dans l'une quelconque des revendications 1-3, en proportion pondérale de 1,0-100%.

10. Médicament suivant la revendication 9, **caractérisé en ce qu'il** est choisi dans le groupe comprenant les lotions, les fluides, les émulsions huile-dans-eau, les crèmes, les gels, les shampooings, les baumes, les conditionneurs, les savons, les pommades, les pâtes, les suspensions et les solutions.

11. Médicament suivant la revendication 10, **caractérisé en ce qu'il** est choisi dans le groupe comprenant la crème, le shampooing, la suspension et la lotion.

12. Méthode de préparation de la composition pharmaceutique suivant les revendications 1-3, **caractérisée en ce qu'elle** comprend les étapes suivantes :
a) la phase aqueuse et les composants de la phase lipidique sont chauffés séparément à la température de 75-85°C,
b) les deux phases sont combinées et homogénéisées, et
c) la microémulsion obtenue est refroidie à la température ambiante,
d) après le refroidissement, des conservateurs sont ajoutés.

13. Méthode suivant la revendication 12, **caractérisée en ce que** l'homogénéisation à l'étape b) est effectuée en appliquant l'une quelconque des techniques choisie dans le groupe comprenant homogénéisation à grande vitesse/ à cisaillement élevé, homogénéisation par ultrasons et/ou homogénéisation à haute pression.
